# EUROPÄISCHE PATENTANMELDUNG

(11) **EP 0 871 033 A2**
(43) Veröffentlichungstag der Anmeldung: **14.10.1998**
(21) Anmeldenummer: 98104507.3
(22) Anmeldetag: 12.03.1998
(51) Int. Cl.: G01N 33/48, G01N 27/49, G01N 21/17, G01N 37/00

(54) **Teststreifenpackung und Messgerät zur Verwendung einer solchen**

(30) Priorität: 09.04.1997 DE 19714674
(71) Anmelder: LRE Technology Partner GmbH, 80807 München (DE)
(72) Erfinder: Markart, Ernst, 81243 Müchen (DE)
(74) Vertreter: Schaumburg, Thoenes & Thurn

(57) **Zusammenfassung**

Bei einer Teststreifenpackung mit einer Mehrzahl von Teststreifen (20), die jeweils ein mittels einer optischen Meßvorrichtung eines Meßgerätes auswertbares Testfeld (32) haben, und mit einem die Teststreifen (20) aufnehmenden Behälter (12) ist dieser von einer rechteckigen geschlossenen flachen Hülle (12) zur Aufnahme einer rechteckigen Karte (18) gebildet, die aus einer Mehrzahl von Teststreifen (20) besteht, die entlang von Abreißlinien (22) miteinander verbunden sind. Das Meßgerät umfaßt ein Gerätegehäuse (38) mit einer Teststreifenauflage (40) sowie im Gehäuse angeordnet eine Meßoptik, eine Auswerte- und Steuerschaltung und eine Anzeigeeinheit (42), wobei die Teststreifenauflage (40) ein zum Eingriff in eine Aussparung (30) in der Hülle (12) bestimmtes Arretierelement (44) sowie einen Anschlag (46) hat, der relativ zur Meßoptik so positioniert ist, daß bei Anlage einer vorgegebenen Stelle eines Teststreifens (20) an dem Anschlag (46) das Testfeld (32) dieses Teststreifens (20) im Bereich des Meßstrahles der Meßoptik liegt.

## Beschreibung

Die Erfindung betrifft eine Teststreifenpackung mit einer Mehrzahl von Teststreifen, die jeweils ein mittels einer optischen oder amperometrischen Meßvorrichtung eines Meßgerätes auswertbares Testfeld haben, und mit einem die Teststreifen aufnehmenden Behälter.

Teststreifen der vorstehend genannten Art dienen beispielsweise dazu, um rasch und auf einfache Weise die Konzentration bestimmter Substanzen in Körperflüssigkeiten zu bestimmen, beispielsweise den Blutzuckergehalt. Diabetiker müssen den Blutzuckergehalt unter Umständen mehrfach am Tage bestimmen und hierzu ein Meßgerät und etliche andere Utensilien mit sich führen, u.a. einen Behälter oder ein Magazin für Teststreifen. Diese Teststreifen müssen innerhalb einer bestimmten Zeit nach dem erstmaligen Öffnen des Behälters verbraucht werden, da sonst die Chemikalien im Testfeld des Teststreifens sich so verändern, daß möglicherweise emne korrekte Konzentrationsmessung nicht mehr erfolgen kann. Bisher ist es üblich, daß der Patient ein kleines Döschen mit einer bestimmten Anzahl von Teststreifen mit sich führt.

Der Erfindung liegt die Aufgabe zugrunde, eine Teststreifenpackung der eingangs genannten Art anzugeben, die bequem und platzsparend mitgeführt werden kann.

Diese Aufgabe wird erfindungsgemäß dadurch gelöst, daß der Behälter von einer rechteckigen, geschlossenen flachen Hülle zur Aufnahme einer rechteckigen Karte gebildet ist, die aus einer Mehrzahl von Teststreifen besteht, die entlang von Abreißlinien miteinander verbunden sind.

Eine solche durch die Hülle geschützte Teststreifenkarte kann bequem mitgeführt werden und sogar, wie dies im folgenden noch erläutert wird, in das Meßgerät eingelegt werden, so daß die Teststreifenpackung nicht als separates Teil neben dem Meßgerät mitgeführt werden muß. Der jeweils benötigte Teststreifen wird vor oder nach Gebrauch von der Teststreifenkarte abgetrennt.

Vorzugsweise hat die Hülle einen Hauptabschnitt und einen von diesem entlang einer vorbereiteten Trennlinie abreißbaren Kopfabschnitt, wobei die Lage der parallel zu den Abreißlinien verlaufenden Trennlinie so gewählt ist, daß nach dem Abtrennen des Kopfabschnittes von dem Hauptabschnitt das Testfeld des ersten faßbaren Teststreifen der Teststreifenkarte noch im Hauptabschnitt liegt. Auch nach dem Öffnen der Hülle ist somit das Testfeld gegen Umwelteinflüsse geschützt, bis der jeweils benötigte Teststreifen aus der Hülle herausgezogen wird.

Für das Einlegen der Teststreifenpackung in das Meßgerät ist es zweckmäßig, wenn in einem Randbereich des Hauptabschnittes der Hülle mindestens eine zum Eingriff eines Arretierelementes des Meßgerätes bestimmte Aussparung so angeordnet ist, daß sie den Teststreifenaufnahmeraum der Hülle nicht berührt oder schneidet. Damit kann die Teststreifenkarte bzw. der jeweils benötigte Teststreifen aus der Hülle herausgezogen werden, wobei die Hülle selbst in dem Meßgerät festgehalten wird.

Die Trennlinie an der Hülle kann beispielsweise durch einander zugeordnete Kerben an den Langsrändern der Hülle vorgegeben sein, um so das Abreißen des Kopfabschnittes von dem Hauptabschnitt an einer vorgegebenen Stelle zu erleichtern.

Die Abreißlinie zwischen einander benachbarten Teststreifen umfaßt vorzugsweise jeweils einen länglichen mittleren Schlitz und Perforationsabschnitte, welche sich jeweils von den Schlitzenden zu den Längsrändern der Karte erstrecken, um so das Abreißen eines gerade benötigten oder bereits verbrauchten Teststreifens von der Teststreifenkarte zu erleichtern.

Ein erfindungsgemäßes Meßgerät zur Verwendung einer vorstehen beschriebenen Teststreifenpackung und zum optischen oder amperometrischen Auswerten des Testfeldes eines Teststreifens umfaßt in bekannter Weise ein Gerätegehäuse mit einer Teststreifenauflage sowie im Gehäuse angeordnet eine Meßoptik bzw. Kontaktierungsvorrichtung, eine Auswert- und Steuerschaltung und eine Anzeigeeinheit. Die Teststreifenauflage ist erfindungsgemäß zur Aufnahme einer Teststreifenpackung der oben beschriebenen Art ausgebildet und hat ein zum Eingriff in eine Aussparung in der Hülle bestimmtes Arretierelement sowie einen Anschlag, der relativ zur Meßoptik bzw. Kontaktierungsvorrichtung so positioniert ist, daß bei Anlage einer vorgegebenen Stelle des Teststreifens an dem Anschlag das Testfeld dieses Teststreifen im Bereich des Meßstrahles der Meßoptik bzw. Kontaktierungsvorrichtung liegt. Durch das Arretierelement wird also die Hülle auf der Teststreifenauflage festgehalten, wenn ein Teststreifen aus der Packung herausgezogen wird. Wenn der Teststreifen bis zu dem genannten Anschlag aus der Hülle herausgezogen wird, ist gleichzeitig sichergestellt, daß dann das Testfeld über der Meßoptik liegt.

Vorzugsweise ist gegenüber der Teststreifenauflage eine in Richtung auf diese vorgespannte Dichtleiste derart angeordnet, daß sie den Randbereich der offenen Seite des Hauptabschnittes der Hülle gegen die Teststreifenauflage drückt. So lange der Teststreifen nicht benutzt wird, liegt das Testfeld innerhalb der Hülle. Diese wird durch die Dichtleiste zugedrückt, so daß das Testfeld gegenüber Umwelteinflüssen, insbesondere gegenüber der Luftfeuchtigkeit geschützt ist. Erst unmittelbar vor der Messung wird der Teststreifen so weit herausgezogen, daß das Testfeld über der Meßoptik liegt.

Die Dichtleiste kann beispielsweise an einer Klappe angeordnet sein, die um eine zur Teststreifenauflage parallele Schwenkachse an dem Gehäuse schwenkbar gelagert ist, so daß die Teststreifenpackung bequem auf die Teststreifenauflage aufgelegt werden kann. Nach dem Schließen der Klappe hält die Dichtleiste die Teststreifenpackung auf der Teststreifenauflage fest und die Hülle geschlossen.

Um das Abreißen des Teststreifens von der Teststreifenkarte zu erleichtern, kann an dem Gehäuse ein eine Trennkante aufweisendes Trennelement derart angeordnet sein, daß es zwischen einer der Auflageflache fernen ersten Stellung und einer der Auflagefläche nahen zweiten Stellung verstellbar ist, in der die Trennkante parallel zu einer Abreißlinie der Teststreifenkarte verläuft. Vorzugsweise ist das Trennelement so ausgebildet, daß es in den oben beschriebenen Schlitz einer Abreißlinie eingreifen kann. Damit hat das Trennelement neben der Abreißfunktion noch die Aufgabe, die Teststreifenkarte in einer bestimmten Position auf der Teststreifenauflage zu fixieren. Der für die Messung gerade benötigte Teststreifen befindet sich damit zwischen der Trennkante des Trennelementes und dem Anschlag auf der Teststreifenauflage in einer exakt vorgegebenen Stellung und wird durch das Trennelement sowie dadurch in dieser Stellung festgehalten, daß er noch mit der von der Dichtlippe gegen die Teststreifenauflage gedrückten Teststreifenkarte verbunden ist.

Vorzugsweise ist das Trennelement an einem schwenkbaren Hebel, beispielsweise der die Dichtleiste tragenden Klappe angeordnet.

Weitere Merkmale und Vorteile der Erfindung ergeben sich aus den weiteren Unteransprüchen und der folgenden Beschreibung, welche in Verbindung mit den beigefügten Zeichnungen die Erfindung anhand eines Ausführungsbeispieles erläutern. Es zeigen:
- Fig. 1: eine schematische perspektivische Ansicht einer Teststreifenpackung,
- Fig. 2: eine der Fig. 1 entsprechende Ansicht einer Teststreifenpackung nach dem Abreißen des Kopfabschnittes,
- Fig. 3: eine schematische perspektivische Ansicht eines erfindungsgemäßen für eine optische Auswertung bestimmten Meßgerätes zur Verwendung einer in den Figuren 1 und 2 dargestellten Teststreifenpackung mit geöffnetem Deckel,
- Fig. 4: eine der Fig. 3 entsprechende Ansicht des Meßgerätes mit geschlossenem Deckel,
- Fig. 5: eine schematische perspektivische Ansicht eines erfindungsgemäßen für eine amperometrische Auswertung bestimmten Meßgerätes,
- Fig. 6: eine der Fig. 5 entsprechende Ansicht des Meßgerätes mit geschlossenem Deckel und
- Fig. 7: eine schematische Draufsicht auf einen Teil der Teststreifenauflage einer abgewandelten Ausführungsform des erfindungsgemäßen Meßgerätes.

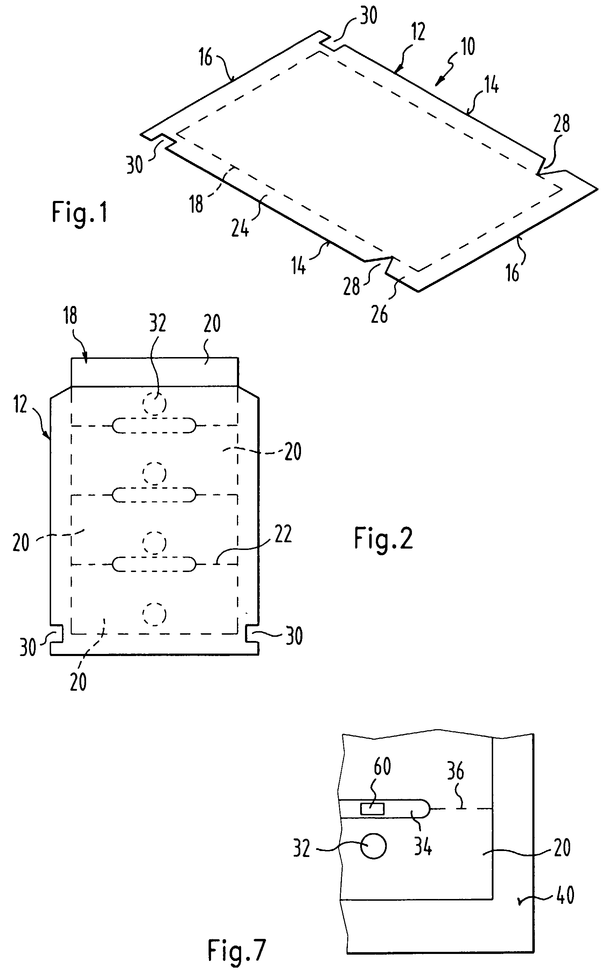

In Fig. 1 ist eine allgemein mit 10 bezeichnete Teststreifenpackung dargestellt. Sie umfaßt eine rechteckige flache Hülle oder Tüte 12, die entlang ihren Längsrändern 14 und ihren Querrändern 16 geschlossen ist. Sie besteht aus zwei Materialbahnen oder einer gefalteten Materialbahn, wobei die offenen Längs- und Querränder 14 bzw. 16 verklebt oder verschweißt sind und die Materialbahnen luft- und feuchtigkeitsundurchlässig sind.

Die Hülle 12 dient zur Aufnahme einer gestrichelt angedeuteten rechteckigen Teststreifenkarte 18 mit einer Mehrzahl von Teststreifen 20, die entlang Abreißlinien 22 miteinander verbunden sind. Die Teststreifenkarte 18 wird weiter unten noch näher erläutert.

Die Hülle 12 umfaßt einen Hauptabschnitt 24 und einen von diesem abtrennbaren Kopfabschnitt 26. Die Trennlinie, entlang der der Kopfabschnitt 26 von dem Hauptabschnitt 24 abgetrennt wird, ist durch zwei einander gegenüberliegende Kerben 28 in den Längsrändern 14 der Hülle 12 markiert.

Nahe dem Ende der Hülle 12, das dem Kopfabschnitt 26 entgegengesetzt ist, sind in den Längsrändern 14 zwei rechteckige Aussparungen 30 ausgebildet, deren Funktion weiter unten noch anhand der Beschreibung des Meßgerätes naher erläutert wird.

Gemäß Fig. 2 hat jeder Teststreifen 20 ein Testfeld 32, auf das eine Körperflüssigkeit, beispielsweise ein Tropfen Blutes aufgetropft werden kann und das anschließend in an sich bekannter Weise optisch analysiert wird. Wird der Kopfabschnitt 26 von dem Hauptabschnitt 24 der Hülle 12 abgerissen, so ragt der erste Teststreifen 20 der Teststreifenkarte 18 gerade so weit aus dem Hauptabschnitt 24 der Hülle 12 heraus, daß er erfaßt werden kann (Fig. 2). Das Testfeld 32 liegt jedoch noch geschützt in dem Hauptabschnitt 24 der Hülle 12.

Jede Abreißlinie 22 zwischen zwei Teststreifen 20 besteht aus einem mittleren Längsschlitz 34 und Perforationslinien 36, die sich von den Schlitzenden zu den Längsrändern 14 hin erstrecken.

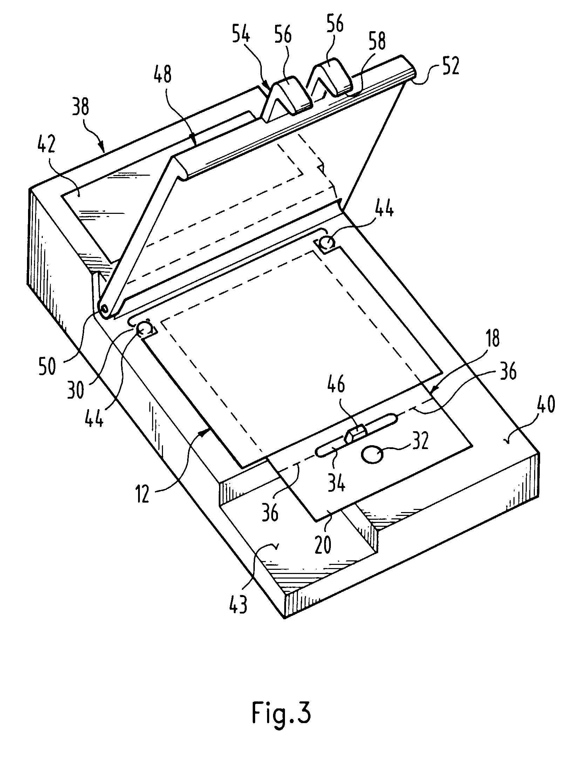

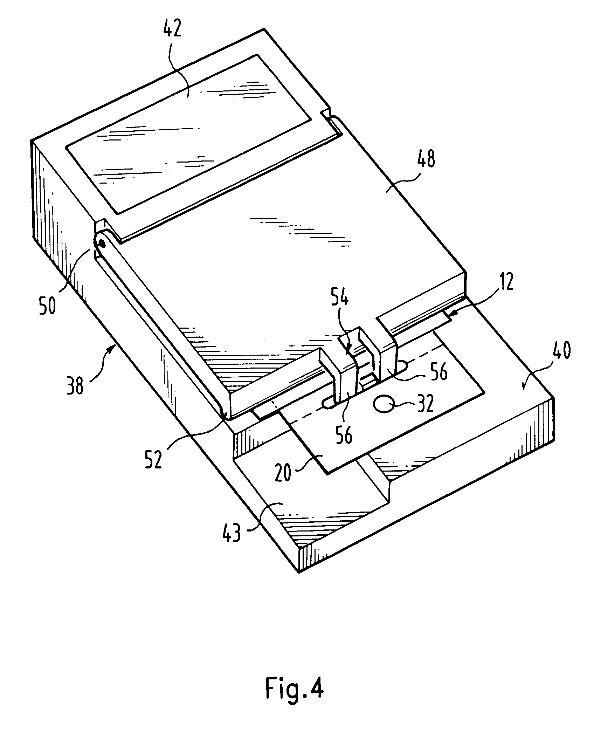

Anhand der Figuren 3 und 4 wird nun ein Meßgerät zur Verwendung der vorstehend beschriebenen Teststreifenpackung 10 erläutert. Das Meßgerät umfaßt ein allgemein mit 38 bezeichnetes Gehäuse mit einer Auflagefläche 40 für die Teststreifenpackung 10. Das Gehäuse enthält in an sich bekannter Weise eine Meßoptik und eine Auswerte- und Steuerschaltung, die beide nicht dargestellt sind, sowie eine Anzeigeeinheit 42. Der Meßstrahl der Meßoptik tritt durch eine in der Auflagefläche 40 ausgebildete Öffnung unterhalb des Testfeldes 32 des in den Figuren 3 und 4 dargestellten, vollständig aus der Hülle 12 herausgezogenen Teststreifens 20. In der Teststreifenauflage 40 ist eine Griffmulde 43 ausgebildet, so daß die Teststreifen leichter erfaßt werden können.

Wie man in Fig. 3 erkennt, ist die Teststreifenpackung 10 so auf die Auflagefläche 40 aufgelegt, daß Arretierzapfen 44, die nahe der Anzeigeeinheit 42 auf der Auflageflache 40 vorgesehen sind, in die rechteckigen Kerben 30 in der Hülle 12 eingreifen. Dadurch ist die Teststreifenpackung 10 auf der Auflagefläche 40 fixiert. Die Hülle 12 bleibt an ihrem Platz, wenn die Teststreifenkarte 18 aus der Hülle 12 herausgezogen wird, bis eine an der Teststreifenauflage 40 angeordnete Nase 46 durch den Schlitz 34 hinter dem ersten herausgezogenen Teststreifen 20 treten kann, wie dies in Fig. 3 dargestellt ist. In dieser Stellung, die auch in der Figur 4 wiedergegeben ist, liegt das Testfeld 32 exakt über der Durchtrittsöffnung für den Meßstrahl.

An dem Gehäuse 38 ist eine Klappe oder ein Deckel 48 um eine zur Auflagefläche 40 parallele Schwenkachse 50 schwenkbar gelagert. Der Deckel 48 kann somit zwischen der in der Fig. 3 dargestellten ersten Stellung, in der die Auflagefläche 40 frei zugänglich ist, um die Teststreifenpackung 10 aufzulegen bzw. die Hülle 12 von der Auflagefläche 40 abzunehmen, und einer zweiten Stellung verschwenkt werden (Fig. 4), in welcher er die Auflagefläche 40 teilweise abdeckt. Der Deckel 48 kann in diese zweite Stellung durch geeignete nicht dargestellte Mittel vorgespannt sein, so daß er fest an der Auflagefläche 40 anliegt.

An seinem der Schwenkachse 50 fernen freien Rand hat der Deckel 48 eine Dichtlippe 52, die in der zweiten Stellung des Deckels 48 auf der Hülle 12 der Teststreifenpackung 10 nahe dem offenen Rand des Hauptabschnittes 24 anliegt. Die Dichtlippe 52 hält einerseits die Hülle 12 geschlossen und andererseits die Teststreifenpackung 10 fest auf der Auflagefläche 40.

Der Deckel 48 trägt ferner an seinem der Schwenkachse 50 fernen freien Ende zwei L-förmige Elemente 54, deren kurzer freier Schenkel jeweils als Trennelement 56 keilförmig ausgebildet ist mit einer freien Trennkante 58. Die Anordnung der Elemente 54 ist so getroffen, daß beim Zuklappen des Deckels 48 die Trennelemente 56 beiderseits der Nase 46 exakt in den Schlitz 34 der Abreißlinie 22 hinter dem vollständig aus der Hülle 12 herausgezogenen Teststreifen 20 greift. Dadurch wird einerseits die Teststreifenkarte 18 auf der Auflagefläche 40 weiter fixiert. Andererseits wird der in der Hülle 12 verbliebenen Teil der Teststreifenkarte 18 festgehalten, wenn der vollständig herausgezogene Teststreifen 20 nach der Durchführung der Messung abgerissen werden soll. Die Anordnung kann auch so getroffen sein, daß das keilförmige Trennelement in eine nicht dargestellte Aussparung in der Teststreifenauflage gedrückt werden kann und dabei die Teststreifen voneinander trennt.

Eine Bedienungsperson geht bei Gebrauch des Meßgerätes folgendermaßen vor:

Zunächst wird das Gerät in an sich bekannter Weise auf eine Messung vorbereitet. Anschließend wird der Deckel 48 aufgeklappt. Von der Hülle 12 der Teststreifenpackung 10 wird der Kopfabschnitt 26 abgetrennt und der Hauptabschnitt 24 auf die Auflagefläche 40 so gelegt, daß die Arretierzapfen 44 in die Kerben 30 eingreifen. Der aus dem Hauptabschnitt 24 herausragende Teil der Teststreifenkarte 18 liegt griffbereit über der Griffmulde 43. Der Deckel 50 wird nun geschlossen. Damit ist das Gerät beladen und die Teststreifenkarte vor Feuchtigkeit geschützt.

Wenn eine Messung durchgeführt werden soll, wird der Deckel 50 geöffnet und der Teststreifen 20 aus der Hülle 12 soweit herausgezogen, daß die Nase 46 durch den unmittelbar auf den herausgezogenen Teststreifen 20 folgenden Schlitz 34 treten kann. Die Position der Nase 46 ist so gewählt, daß in dieser Stellung der Teststreifenkarte 18 das Testfeld 32, das bisher in der Hülle 12 geschützt war, über der Meßöffnung liegt. In dieser Stellung wird eine Leermessung des Testfeldes 32 ausgeführt. Anschließend wird die zu analysierende Körperflüssigkeit aufgetropft und die eigentliche Messung durchgeführt. Der von der Auswerteschaltung gelieferte Meßwert erscheint in der Anzeigeeinheit 42. Nun wird der Deckel 48 geschlossen, wobei das Trennelement 46 in den Schlitz 34 hinter dem vollständig herausgezogenen Teststreifen 20 eingreift. Der verbrauchte Teststreifen 20 kann abgetrennt werden. Der vorstehend beschriebene Meßvorgang kann wiederholt werden, so lange Teststreifen in der Hülle 12 vorhanden sind. Anschließend muß eine neue Teststreifenpackung 10 eingelegt werden.

Zusätzlich oder alternativ zu der Positionierung des Teststreifens 20 mittels der Nase 46 kann auch mittels der Meßoptik festgestellt werden, ob das Testfeld 32 exakt über der Meßöffnung liegt. Zu dem gleichen Zweck kann auch eine Zusatzoptik 60 vorgesehen sein (Fig. 7), die entweder beispielsweise eine Durchbrechung wie den Schlitz 34 erfaßt, wenn das Testfeld 32 über der Meßoptik liegt. Dieser Fall ist in Fig. 7 dargestellt. Ebenso kann die Zusatzoptik aber auch auf eine Markierung an dem Teststreifen ansprechen, wobei die Markierung und die Zusatzoptik relativ zu dem Testfeld bzw. der Meßoptik so angeordnet sind, daß beim Erfassen der Markierung durch die Zusatzoptik das Testfeld über der Meßoptik liegt.

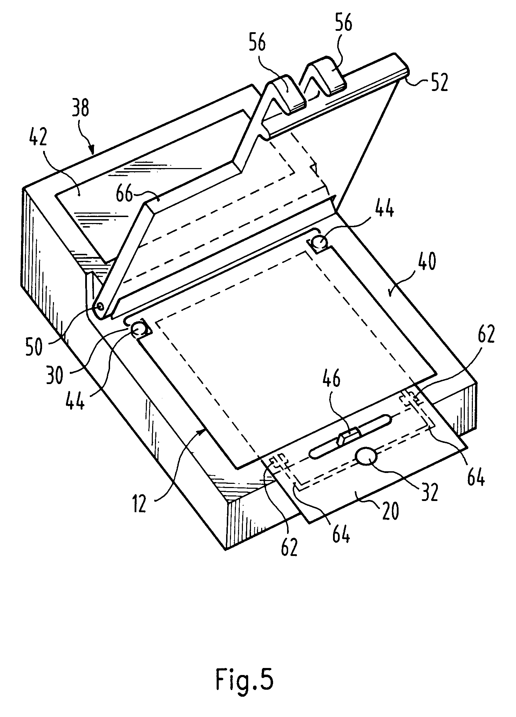

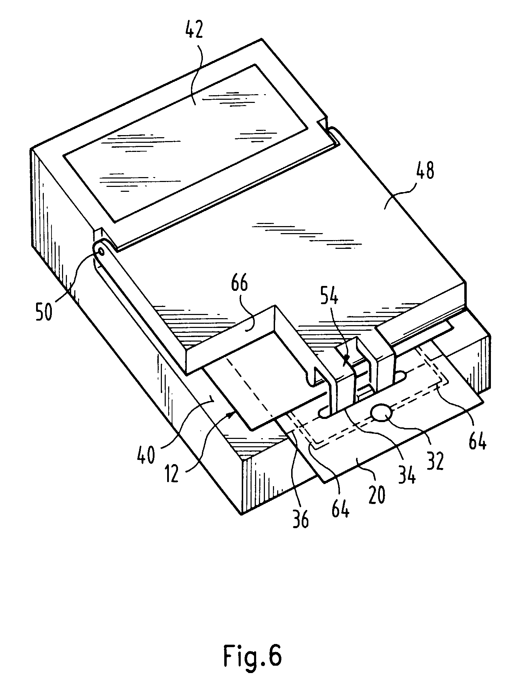

Eine weitere Ausführungsform des erfindungsgemäßen Meßgerätes ist in den Figuren 5 und 6 dargestellt. Das dort abgebildete Meßgerät ist für eine amperometrische Auswertung von Teststreifen geeignet. Gleiche Teile wie bei dem Meßgerät gemäß den Figuren 3 und 4 sind mit gleichen Bezugszeichen versehen und werden nicht noch einmal erläutert.

Der wesentliche Unterschied zu dem Meßgerät gemäß den Figuren 3 und 4 liegt darin, daß das Gerät keine Meßoptik sondern eine Kontaktierungsvorrichtung mit in der Teststreifenauflage 40 angeordneten Kontaktelementen 62 hat, die mit Kontaktbahnen 64 auf den Teststreifen 20 in Kontakt treten können, wenn die Teststreifenkarte 18 auf der Teststreifenauflage 40 aufliegt. Das Gehäuse 38 ist gegenüber dem in den Figuren 3 und 4 dargestellten Gehäuse verkürzt. Die Nase 46 befindet sich an dem vorderen Rand des Gehäuses 38. In dem Deckel 48 ist eine Aussparung 66 vorgesehen, die das Erfassen des aus der Hülle 12 herauszuziehenden Teststreifens 20 erleichtert.

Die Vorbereitung des Meßgerätes erfolgt in der oben beschriebenen Weise. Nachdem der Teststreifen 20 mittels der Nase 46 positioniert wurde, wird die zu analysierende Flüssigkeit auf das Testfeld 32 aufgetropft. Mit Hilfe der Kontaktelemente 62 kann der über das Testfeld 32 dann fließende Strom gemessen werden. Die gemessene Stromstärke kann dann zur Bestimmung der Konzentration der gesuchten Substanz ausgewertet werden. Der aus dem geschlossenen Gerät herausragende Teststreifen 20 kann dann abgerissen oder durch das Eintauchen der Trennelemente 56 in den Schlitz 34 abgetrennt werden.

Auch bei dem Gerät gemäß den Figuren 5 und 6 kann eine Zusatzoptik zur genauen Positionierung des Teststreifens 20 vorgesehen sein.

Bei beiden Ausführungsformen kann die Zusatzoptik und bei der Ausführungsform gemaß den Figuren 3 und 4 auch gegebenenfalls die Meßoptik als Sende- und/oder Empfangseinrichtung ausgebildet sein, um Daten zu übertragen bzw. zu empfangen. So können beispielsweise gespeicherte Meßwerte aus dem Meßgerät ausgelesen werden. Umgekehrt kann das Meßgerät Eichdaten und sonstige Informationen über die zu verwendenden Teststreifen auf diesem Wege erhalten.

## Patentansprüche

1. Teststreifenpackung mit einer Mehrzahl von Teststreifen (20), die jeweils ein mittels einer optischen oder amperometrischen Meßvorrichtung eines Meßgerätes auswertbares Testfeld (32) haben, und mit einem die Teststreifen (20) aufnehmenden Behälter (12), dadurch **gekennzeichnet**, daß der Behälter von einer rechteckigen geschlossenen flachen Hülle (12) zur Aufnahme einer rechteckigen Karte (18) gebildet ist, die aus einer Mehrzahl von Teststreifen (20) besteht, die entlang von Abreißlinien (22) miteinander verbunden sind.

2. Teststreifenpackung nach Anspruch 1, dadurch **gekennzeichnet**, daß die Hülle (12) einen Hauptabschnitt (24) und einen von diesem entlang einer vorbereiteten Trennlinie abreißbaren Kopfabschnitt (26) umfaßt, wobei die Lage der parallel zu den Abreißlinien (22) verlaufenden Trennlinie so gewählt ist, daß nach dem Abtrennen des Kopfabschnittes (26) von dem Hauptabschnitt (24) das Testfeld (32) des ersten erfaßbaren Teststreifens (20) der Teststreifenkarte (18) noch im Hauptabschnitt (24) liegt.

3. Teststreifenpackung nach Anspruch 2, dadurch **gekennzeichnet**, daß in einem Randbereich des Hauptabschnittes (24) der Hülle (12) mindestens eine zum Eingriff eines Arretierelementes (44) des Meßgerätes bestimmte Aussparung (30) so angeordnet ist, daß sie den Teststreifenaufnahmeraum der Hülle nicht berührt oder schneidet.

4. Teststreifenpackung nach Anspruch 2 oder 3, dadurch **gekennzeichnet**, daß die Trennlinie durch einander zugeordnete Kerben (28) in den Längsrandbereichen (14) der Hülle (12) vorgegeben ist.

5. Teststreifenpackung nach einem der Ansprüche 1 bis 4, dadurch **gekennzeichnet**, daß die Abreißlinien (22) jeweils einen länglichen mittleren Schlitz (34) und Perforationsabschnitte (36) umfassen, welche sich jeweils von den Schlitzenden zu den Längsrändern (12) der Teststreifenkarte (18) erstrecken.

6. Meßgerät zum optischen oder amperometrischen Auswerten eines Testfeldes (32) eines Teststreifens (20), umfassend ein Gerätegehäuse (38) mit einer Teststreifenauflage (40) sowie im Gehäuse angeordnet eine Meßoptik bzw. Kontaktierungsvorrichtung, eine Auswerte- und Steuerschaltung und eine Anzeigeeinheit (42), dadurch **gekennzeichnet**, daß die Teststreifenauflage (40) zur Aufnahme einer Teststreifenpackung (10) nach einem der Ansprüche 3 bis 5 ausgebildet ist und ein zum Eingriff in eine Aussparung (30) in der Hülle (12) bestimmtes Arretierelement (44) sowie einen Anschlag (46) hat, der relativ zur Meßoptik bzw. Kontaktierungsvorrichtung so positioniert ist, daß bei Anlage einer vorgegebenen Stelle eines Teststreifens (20) an dem Anschlag (46) das Testfeld (32) dieses Teststreifens (20) im Bereich des Meßstrahles der Meßoptik bzw. Kontaktierungsvorrichtung liegt.

7. Meßgerät nach Anspruch 6, dadurch **gekennzeichnet**, daß gegenüber der Teststreifenauflage (40) eine in Richtung auf diese vorgespannte Dichtleiste (52) derart angeordnet ist, daß sie den Randbereich der offenen Seite des Hauptabschnittes (24) der Hülle (12) gegen die Teststreifenauflage (40) drückt.

8. Meßgerät nach Anspruch 7, dadurch **gekennzeichnet**, daß die Teststreifenpackung (10) und die Dichtleiste (52) relative zueinander so angeordnet sind, daß in einer Transportlage des Meßgerätes ein Griffabschnitt eines Testreifens vor der Dichtleiste (52) und das Testfeld (32) hinter der Dichtleiste (52) liegt.

9. Meßgerät nach Anspruch 7 oder 8, dadurch **gekennzeichnet**, daß die Dichtleiste (52) an einer Klappe (48) angeordnet ist, die um eine zur Teststreifenauflage (40) parallele Schwenkachse (50) an dem Gehäuse (38) schwenkbar gelagert ist.

10. Meßgerät nach einem der Ansprüche 6 bis 9, dadurch **gekennzeichnet**, daß an dem Gehäuse (38) mindestens ein eine Trennkante (58) aufweisendes Trennelement (56) derart angeordnet ist, daß es zwischen einer der Teststreifenauflage (40) fernen ersten Stellung und einer der Teststreifenauflage (40) nahen zweiten Stellung verstellbar ist, in der die Trennkante (58) parallel zu einer Abreißlinie (22) der Teststreifenkarte (18) verläuft.

11. Meßgerät nach Anspruch 10, dadurch **gekennzeichnet**, daß das Trennelement (56) zum Eingriff in einen Schlitz (34) einer Abreißlinie (22) ausgebildet ist.

12. Meßgerät nach Anspruch 10 oder 11, dadurch **gekennzeichnet**, daß das Trennelement (56) an einem Schwenkhebel (48) angeordnet ist, dessen Schwenkachse (50) parallel zur Teststreifenauflage (40) und zur Trennkante (58) gerichtet ist.

13. Meßgerät nach den Ansprüchen 7 und 12, dadurch **gekennzeichnet**, daß das Trennelement (56) an der die Dichtleiste tragenden Klappe (48) angeordnet ist.

14. Meßgerät nach einem der Ansprüche 11 bis 13, dadurch **gekennzeichnet**, daß das Trennelement keilförmig ausgebildet ist, so daß es beim Eingriff in den Schlitz (34) einer Abreißlinie (22) die an dieser aneinandergrenzenden Teststreifen trennt.

15. Meßgerät nach einem der Ansprüche 6 bis 14, dadurch **gekennzeichnet**, daß es eine Zusatzoptik (60) zum Erfassen einer Markierung oder Durchbrechung an der Teststreifenkarte (18) hat, um die korrekte Lage des Teststreifens (20) in dem Meßgerät zu erfassen.

16. Meßgerät nach einem der Ansprüche 6 bis 15, dadurch **gekennzeichnet**, daß die Meßoptik oder die Zusatzoptik (60) als Sende- und/oder Empfangseinrichtung zur optischen Datenübertragung ausgebildet sind.

17. Meßgerät nach Anspruch 16, dadurch **gekennzeichnet**, daß die Zusatzoptik (60) so angeordnet ist, daß sie bei eingelegter Teststreifenpackung durch den Schlitz (34) in der Abreißlinie (22) hindurch Daten übermitteln oder empfangen kann.

18. Meßgerät nach einem der Ansprüche 6 bis 17, dadurch **gekennzeichnet**, daß es eine Transportvorrichtung mit Transportelementen hat, die zum Eingriff mit einer Tranportperforation an der Teststreifenkarte bestimmt sind, um diese um einen vorgegebenen Betrag vorzuschieben.
